# EUROPEAN PATENT APPLICATION

(11) **EP 3 982 324 A1**
(43) Date of publication of application: **13.04.2022**
(21) Application number: 20200938.7
(22) Date of filing: 09.10.2020
(51) Int. Cl.: G06T 7/00, G06T 7/11, G06T 7/60, G06T 11/60, G16H 30/40

(54) **GENERATING A SYTHETIC IMAGE FOR A BLOOD VESSEL LESION CLASSIFIER**

(71) Applicant: Koninklijke Philips N.V., 5656 AG Eindhoven (NL)
(72) Inventor: NICKISCH, Hannes, 5656 AE Eindhoven (NL); SCHNELLBÄCHER, Nikolas David, 5656 AE Eindhoven (NL); GRASS, Michael, 5656 AE Eindhoven (NL); FRANZ, Astrid Ruth, 5656 AE Eindhoven (NL); WISSEL, Tobias, 5656 AE Eindhoven (NL)
(74) Representative: Philips Intellectual Property & Standards

(57) **Abstract**

A mechanism for generating synthetic images of blood vessels having lesions, for training a machine-learning method that analyzes lesions in images of blood vessels. The proposed mechanism obtains an image of (healthy) blood vessels and performs image segmentation to determine characteristics of the blood vessels in the image. The determined characteristics are used to generate a set of one or more lesions for the blood vessels. The generated set of one or more lesions and the original image are then combined to generate a synthesized image of the blood vessels having (synthetic) lesions.

## Description

### FIELD OF THE INVENTION

The present invention relates to the field of predictive learning models, and in particular to predictive learning models for images of blood vessels.

### BACKGROUND OF THE INVENTION

The advent of machine-learning methods, such as those that employ neural networks, has improved the accuracy of analyzing medical images. However, modern machine-learning methods require a large amount of reliable ground-truth data for accurate training. As well as quantity and reliability, there are a number of other desirable characteristics for ground-truth datasets, such as high levels of accuracy, detail, variance and/or coverage (of different possible options for the medical image).

At present, it is difficult to obtain ground-truth datasets that meet all of these requirements. This is primarily due to difficulties in obtaining a suitable range of ground-truth data (e.g. due to lack of real-world examples) and the significant level of clinical time, expertise and experience required to ensure that any provided ground-truth data is accurate.

One area of medical image processing (with machine-learning methods) that faces the above-described problem is in applying machine-learning models to images of blood vessels, and in particular to determining the presence (and characteristics) of lesions within blood vessels.

There is therefore a desire to improve the availability of ground-truth data for training a machine-learning method suitable for analyzing images to identify, classify or otherwise determine characteristics of blood vessel lesions in the images.

### SUMMARY OF THE INVENTION

The invention is defined by the claims.

According to examples in accordance with an aspect of the invention, there is provided a computer-implemented method for generating a synthetic image for training a machine-learning method for assessing lesions in blood vessels of an image.

The computer-implemented method comprises: obtaining an image of blood vessels in a region of interest of a subject; performing image segmentation on the image to identify one or more parameters of the blood vessels from the image; synthesizing a set of one or more lesions for one or more of the blood vessels of the image by processing the identified one or more parameters of the blood vessels; and generating the synthetic image, for training a machine-learning method for assessing lesions in blood vessels of an image, by combining the image and the synthesized set of one or more lesions for one or more of the blood vessels contained in the image; and generating one or more annotations for the synthetic image, based upon the characteristics of the set of one or more synthetic lesions in the synthetic image.

The disclosure proposes a mechanism for synthesizing lesions for one or more blood vessels depicted in an image. The image is segmented, e.g. processed or analyzed, to identify one or more parameters of the blood vessel(s). The identified parameters are then processed to generate one or more lesions for the blood vessel(s). Subsequently, the image and the generated lesion(s) are combined to thereby generate an image having synthesized lesions.

The present disclosure provides a new approach for generating a synthetic image of blood vessels having lesions. Rather than modifying an existing image of blood vessels with lesions (e.g. transforming, rotating or skewing an existing image), brand new synthetic images can be generated from images of "health" blood vessels - e.g. blood vessels absent of lesions.

In particular, the disclosure proposes the generation of synthetic lesions for insertion into an existing image. By basing the synthetic lesions on characteristics of blood vessels in an existing image, the synthetic image can accurately resemble a real-life example of a blood vessel having a lesion.

The present disclosure recognizes that synthetic lesions for blood vessels can be accurately generated based on (accurate) segmentation of an image of blood vessels. In particular, the segmentation can identify characteristics of the blood vessels, which can define a possible shape/outline and/or position for synthetic lesions within the image.

Preferably, the obtained image comprises an image of blood vessels having no lesions, or a lesion burden below a predetermined threshold. The image may, for instance, be selected from historical data (identifying a lesion-less image of blood vessels) or by a user.

The image may comprise a computed tomography image. Computer tomography an image is particularly useful for identifying blood vessels, so that the present invention is particularly advantageous when used to generate a synthetic computed tomography image of blood vessels having lesions.

In some examples, the one or more parameters of the blood vessels includes a position and/or shape of at least one blood vessel in the image.

In some examples, the one or more parameters of the blood vessels includes at least a position of at least one blood vessel in the image; and the step of synthesizing a set of one or more lesions comprises defining a location for each lesion, with respect to the image, based on the identified position of at least one blood vessel in the image.

Use of the determined position of the blood vessels facilitates accurate positioning of a synthetic lesion in a realistic position with respect to the blood vessel. In particular, a synthetic lesion can be accurately positioned within a blood vessel if the location of the blood vessel is known.

The step of defining a location for each lesion may comprise defining the location of each lesion using a partially stochastic generation procedure. A stochastic generation procedure allows the position of the lesions to be pseudorandomized, facilitating the generation of a broader range of synthetic images, e.g. to reduce the likelihood that repeated generation of synthetic images will generate synthetic lesions at a same position, which increases the likelihood that a machine-learning method (trained on synthetic images) will suffer from overfitting.

In some embodiments, the one or more parameters of the blood vessels includes at least a shape of at least one blood vessel in the image; and the step of synthesizing a set of one or more lesions comprises defining a shape for each lesion based on the identified shape of the at least one blood vessel in the image.

By defining a shape for each lesion based on the shape of the blood vessel, the synthesized lesions are more likely to accurately represent or model "true" or real-life lesions. This means that a machine-learning method, trained using such synthetic images, will be more accurate when processing real-life examples of an image.

In particular, the shape for each lesion may be configured to match or mirror a shape of a wall or exterior of (part of) a blood vessel.

Optionally, the step of generating the synthetic image comprises: generating, for each lesion, a motion vector field, which imitates the growth of the respective lesion, based on parameters of the shape of the respective lesion; and generating the synthetic image by processing the image, the shape and the motion vector field for each lesion in the synthesized set of one or more lesion.

In particular, the method may comprise using the motion vector field, of each lesion in the synthesized set of one or more lesion, to modify the values of pixels or voxels of the image at the position of each of the synthesized set of one or more lesions, to thereby generate a modified image; and generating the synthetic image by processing the modified image and the shape of each lesion in the synthesized set of one or more lesion

The use of a motion vector field to modify the values of pixels of the (original) image) means that the synthesized image more closely resembles a "true" image of blood vessels having lesions.

In some examples, the step of using the motion vector field to modify the values of pixels or voxels comprises, for each motion vector field, locally shifting the pixels or voxels of the image at the position of the motion vector field on values of the motion vector field.

Locally shifting the pixels or voxels means that image data of the original image is not lost when generating the synthetized image, but is rather moved to reflect the likely effect that the synthesized lesion would have on a blood vessel if it were a real lesion in the blood vessel of the original image. Thus, the synthesized image data more closely resembles a "true" image of blood vessels having one or more lesions, improving the training dataset for the machine-learning method.

The step of generating the synthetic image optionally comprises texturizing the synthesized set of one or more lesions using a texture pattern. Texturizing synthesized lesions results in the lesions present in the synthesized an image more closely resembling real-life lesions. This improves the accuracy and relevance of the machine-learning method.

The texture pattern may be selected based upon a desired use case for the synthesized image, e.g. to texturize a synthesized lesion based on the desired use of the machine-learning method (e.g. a desired classification task for the machine-learning method).

Preferably, the blood vessels are coronary arteries. There is a particular lack of available ground-truth data for coronary arteries, so that the use of the synthetic images in machine-learning methods for coronary arteries is particularly advantageous when implemented in this field.

There is also proposed a computer-implemented method of training a machine-learning method for assessing lesions in blood vessels of an image. The computer-implemented method comprises: obtaining one or more synthetic images, and one or more annotations for each synthetic image, generated using any previously described method; and training a machine-learning method for assessing lesions in blood vessels of an image using the obtained synthetic image.

There is also proposed a computer program product comprising computer program code means which, when executed on a computing device having a processing system, causes the processing system to perform all of the steps of any herein described method.

There is also proposed a processing system for generating a synthetic image for training a machine-learning method for assessing lesions in blood vessels of an image. The processing system is configured to: obtain an image of blood vessels in a region of interest of a subject; perform image segmentation on the image to identify one or more parameters of the blood vessels from the image; synthesize a set of one or more lesions for one or more of the blood vessels of the image by processing the identified one or more parameters of the blood vessels; generate the synthetic image, for training a machine-learning method for assessing lesions in blood vessels of an image, by combining the image and the synthesized set of one or more lesions for one or more of the blood vessels contained in the image; and generate one or more annotations for the synthetic image, based upon the characteristics of the set of one or more synthetic lesions in the synthetic image.

There is also proposed a processing system for training a machine-learning method for assessing lesions in blood vessels of an image, processing system being configured to: obtain one or more synthetic images, and one or more annotations for each synthetic image, generated using a previously described method and train a machine-learning method for assessing lesions in blood vessels of an image using the obtained synthetic image(s).

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a method according to an embodiment;
Figure 2 illustrates an approach for synthesizing a lesion;
Figure 3 illustrates examples of synthesized images;
Figure 4 illustrates another example of a synthesized image;
Figure 5 illustrates a method according to an embodiment; and
Figure 6 illustrates a processing system according to an embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for generating synthetic images of blood vessels having lesions, for training a machine-learning method that analyzes lesions in an image of blood vessels. The proposed mechanism obtains an image of (healthy) blood vessels and performs image segmentation to determine characteristics of the blood vessels in the image. The determined characteristics are used to generate a set of one or more lesions for the blood vessels. The generated set of one or more lesions and the original an image are then combined to generate synthesized an image of the blood vessels having (synthetic) lesions.

Embodiments are based on the realization that accurate segmentation of blood vessels facilitates the generation of accurate synthetic lesions. In particular, accurate models of a synthetic lesion can be generated as the features of the blood vessels (into which the synthetic lesions are to be inserted) can be accurately determined.

Thus, rather than generating new synthetic images by modifying existing images of blood vessels having lesions, new synthetic images can be generated by the insertion of synthetic lesions into an image of healthy blood vessels.

Generally, the present disclosure provides a synthetic lesion growth/generation model that facilitates the generation of generate artificial/synthetic lesions in/from imaging data, such as a CT image or CT scan. Given an accurate segmentation of the blood vessel(s) in the imaging data, (geometric) properties or characteristics of the blood vessel(s) such as radii, angles, lengths and so on, all of which may vary along the length of the blood vessel(s), can be used to generate a shape of the lesion. From the shape, a motion vector field (MVF), or other growth approach, can be used to mimic the lesion growth process.

Using the determined shape and the growth model, the lesion can be inserted into the image of a blood vessel using a texture module, to generate a suitable synthetic image for training a machine-learning algorithm. All these parameters can be tailored to specific needs of the machine-learning algorithm or requirements at the specific site.

The proposed approach can be used to generate synthetic images of any region of interest of the user having synthetic lesions. For instance, synthetic images could be generated that emulates a plaque load in peripheral vascular systems (for use in training a plaque load estimators for valvular treatment planning). As another example, a synthetic image could be generated that emulates a carotid plaque or aneurysm in a neurovascular system, e.g. for use in training a machine-learning method for assessing a neurovascular system.

In the context of the present disclosure, a lesion is considered to be any region of a blood vessel that undergoes growth to disrupt the normal operation of the blood vessel or increase the likelihood of atherosclerosis, stroke or any other condition resulting from reduced blood flow. Examples include: a growth, plaque, a swelling, an aneurysm, a bulge, a thickening, a calcification, an accumulation of material (e.g. fat, cholesterol or the like), a tumor, a lump, a malignancy, an ulcer, an abscess, polyps and so on.

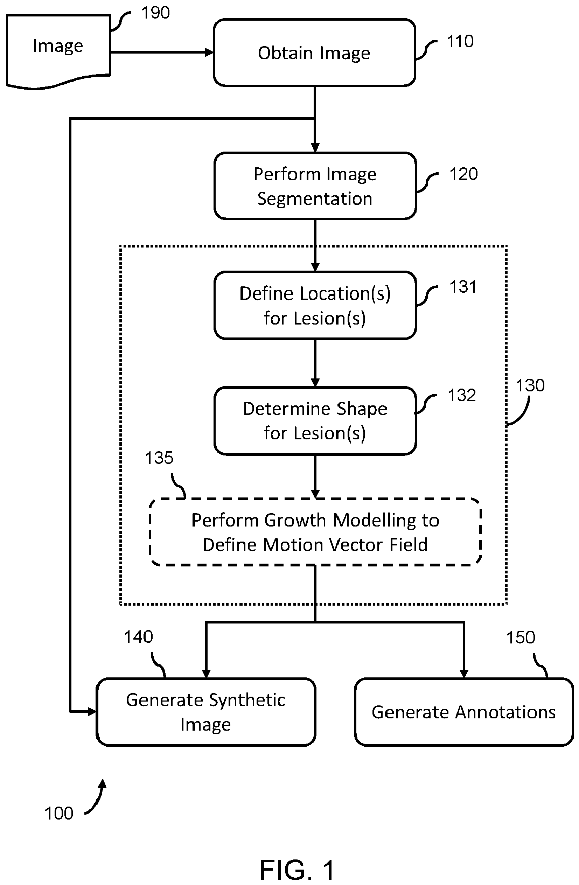

Figure 1 illustrates a computer-implemented method 100 according to an embodiment of the invention.

The computer-implemented method 100 comprises a step 110 of obtaining an image 190 of blood vessels in a region of interest of a subject.

The image 190 may, for instance, comprise a (medical) image of the subject, such as a computed tomography (CT) image. The image may therefore comprise pixels or voxels having values for an image of the region of interest. Preferably, the image 190 is a 3D image of the region of interest, although embodiments may also be used with a 2D image of the region of interest.

Preferably, the image is an image of a subject having no lesions in the blood vessels in the region of interest. In other words, the image may be an example of a "healthy" image.

The image 190 then undergoes a segmentation process in step 120. The segmentation process identifies characteristics or properties of one or more blood vessels in the image. Mechanisms and approaches for segmenting images are well known to the skilled person.

In particular, the identified characteristics or properties may include at least a location of the blood vessel(s) (i.e. in the image) and/or a shape of the blood vessel(s). The location of the blood vessel(s) may be defined by a position of a centerline of the blood vessel or by a position of the exterior walls of the blood vessel. The shape of the blood vessel may be defined using predefined shape parameters, e.g. a radius/diameter of the blood vessel, a length of the blood vessel, an angle of the blood vessel and so on. In particular examples, the shape of the blood vessel(s) may define an outline of the blood vessel(s) within the image, i.e. identify a segment of the image containing the blood vessel(s).

In particular examples, the segmentation process may identify a location of the centerline of the blood vessel and geometric properties of the blood vessel from the centerline (e.g. radii, angles and lengths) that vary along the center line.

The method 100 then performs a step 130 of generating or synthesizing a set of one or more lesions or growths based on the determined characteristics or properties of the one or more blood vessels.

In this way, a synthetic (or artificial) set of one or more lesions are generated for the image. By basing the synthetic set of lesions on the characteristics of the blood vessels (in the image), the lesions can be refined or tailored to the specific blood vessels depicted in the image, improving the accuracy and relevance of the synthesized lesions.

The number of lesions generated in step 130 may be determined based on a user input (e.g. indicating a desired number of lesions), historical data and/or a pseudorandom process. For instance, a user or historical data may indicate a maximum number of lesions, and a pseudorandom process may select up to the maximum number of lesions using a pseudorandom process, such as a stochastic generation procedure.

Step 130 may comprise a step 131 of defining a location/position for each lesion (with respect to the image) based on a determined location of the blood vessel(s) in the image. In other words, if the segmentation process identifies where the blood vessels are located, a synthetic lesion can be accurately positioned within a blood vessel depicted in an image.

In particularly preferably examples, the location of each lesion is defined using a (partially) stochastic generation procedure. In particular, the location of the blood vessel(s), e.g. parts of the image that correspond to a blood vessel, may be used to define the possible locations for the lesion. Of these potential locations, a lesion location may be selected using a stochastic generation procedure, e.g. to pseudorandomly select a position for the lesion within a blood vessel.

Step 130 may comprise a step 132 of defining a shape for each lesion based on the shape of the blood vessel. The shape of a lesion may define the centerline for the lesion, and/or the exterior extent/bounds or outline of the lesion. The size/shape of the lesion can be selected to follow or geometrically compliment an interior wall shape of the blood vessel (in which the lesion is to be placed). In particular, the centerline for the lesion may follow or geometrically compliment the interior wall shape of the blood vessel.

In particular, the shape may define the (predicted) maximum possible extent for a synthetic lesion with respect to the blood vessel, e.g. a predicted maximum extent to which a lesion will grow with respect to the blood vessel. The shape of the lesion may be defined using shape parameters, which may define the characteristics/extent of the shape with respect to a centerline of the lesion (as identified from the image segmentation process).

The size (e.g. volume or surface area) of the shape for the lesion may be predetermined. For instance, it may be defined based on a user input (defining a size for the shape or an amount of lesion burden within the image) and/or historical data of lesions (e.g. defining examples of sizes for lesions within a particular blood vessel).

In some preferred examples, the size (e.g. volume or surface area) of the shape is selected using a pseudorandom process or a (partially) stochastic generation procedure. For instance, a maximum possible size for the shape may be determined based on a user input (defining a maximum possible size) and/or historical data (providing sample sizes for lesions of a particular blood vessel). The size for the shape may then be selected by a pseudorandom process, e.g. using a stochastic generation procedure, selecting a size for the shape between a minimum size (e.g. 0) and the maximum possible size.

In some examples, the size of the shape is dependent upon the size of the blood vessel. For instance, the size of the shape may be selected to fill a predetermined percentage of a particular blood vessel (or part of a blood vessel), the predetermined percentage being defined based on a user input or historical data.

In particularly preferably embodiments, step 132 may comprise defining a shape for each lesion based on the shape of the blood vessel at a defined location for each lesion, as determined in step 131. In other words, the shape and/or size of the shape for each lesion may be dependent upon the determined location for the lesion. This effectively sets a shape (e.g. the centerline and exterior bounds) and position for the lesion within the image based on data obtained from segmenting the image, e.g. to appropriately place and shape each lesion within a blood vessel.

Again, in some examples, step 132 may comprise using a predetermined size or a pseudorandomly selected size for the shape of each lesion at the defined location within the blood vessel.

In particular, step 132 may comprise defining which parts (e.g. which pixels and/or voxels) of the image represent the exterior bounds of a synthetic lesion. For instance, in one example, the image is a 3D image of the region of interest, step 130 obtains a voxelized and explicit geometric 3D lesion outline from the determined parameters of the blood vessel(s).

In some embodiments, the method further performs a step 135 of generating a motion vector field for each synthetic lesion. This step may be performed by a growth module.

The motion vector field models the growth of the lesion, i.e. mimics the lesion growth process. In particular examples, for each lesion, a smooth motion vector field is obtained that describes the lesion growth. The smooth motion vector field may, for instance, be a vector field that has zero values outside of the exterior bounds of the lesion and, preferably, along a centerline of the lesion.

Thus, step 135 further generates a motion vector field for each lesion, which describes a predict growth of the lesion.

The motion vector field can be generated as the final bounds of the lesion for the image have already been defined (in step 132), and the stages of lesion growth from an initial positon, i.e. the centerline of the lesion, are established in the art, i.e. the process by which a lesion grows are well established. This means that a motion vector field mimicking a predicted growth of a synthesized lesion can be generated with ease.

The method then performs a step 140 of generating the synthetic image, for training a machine-learning method for assessing lesions in blood vessels of an image, by combining the (original) image and the synthesized set of one or more lesions for one or more of the blood vessels contained in the image.

Effectively, step 140 comprises inserting the synthetic set of one or more lesions into the image, to generate synthetic images depicting (real) blood vessels in the region of interest with synthetic lesions located in the blood vessels.

Step 140 may comprise, for example, modifying the image based on generated set of one or more lesions. In particular, if the shape/outline and position of each lesion in the set of one or more lesions (with respect to the image) is known, then the image can be appropriately modified to further include the synthetic lesion(s).

In some examples, the step of generating the synthetic image comprises overlaying the synthesized set of one or more lesions over the image to thereby generate the synthetic image.

In some examples, step 140 includes texturizing the synthetic lesions (when inserting the synthetic lesions into the image). The texture of the synthetic lesions may be based upon a desired type of lesion, e.g. a lesion to be targeted by a particular machine-learning method. In particular, a standard or average texture pattern of a particular types of lesion could be used dependent upon implementation desires, and may be set by an individual or user of the machine-learning method, or may be set automatically (e.g. if a machine-learning method lacks sufficient training data).

For instance, a first texture may be used if there is a desire for a synthetic lesion to represent a wall-thickening of the blood vessel; a second texture may be used if there is a desire for a synthetic lesion to represent a calcification in the blood vessel; and a third texture may be used if there is a desire for a synthetic lesion to represent a spotty calcification in the blood vessel. Other approaches and textures would be apparent depending upon the contextual use of the synthetic image.

In this way, the synthetic image is generated for training a machine-learning method for assessing lesions in blood vessels of an image.

In some embodiments, the texture pattern may be dependent upon imaging parameters or modalities of the image 190. For example, different imaging modalities may result in different textures for lesions identified in the image. As an example, different monoenergetic levels for CT image of a same region will result in different textures for a same lesion. Thus, to make the synthetic image more closely resemble a true or real-life image of a blood vessel with a lesion, the texture for the lesion(s) may depend upon the imaging parameter/modality of the image 190.

In some further examples, step 140 makes use of any motion vector fields generated for each lesion (if generated in step 135).

In particular, step 140 may comprise using the motion vector field, of each lesion in the synthesized set of one or more lesion, to modify the values of pixels or voxels of the image at the position of each of the synthesized set of one or more lesions, to thereby generate a modified image.

For instance, each motion vector field may be used as an optical flow to locally shift the pixels/voxels of the image at the position of the motion vector field. This shifting of the pixels/voxels effectively frees the canvas for insertion of a lesion, without loss of image information about the bounds of blood vessel (as this information will be simply shifted, rather than lost). Moreover, using a motion vector field to locally shift the pixels causes the blood vessels of the image to undergo a simulated shift due to the growth of a lesion (i.e. so that the portions of the "healthy" blood vessel in the image are moved/modified to simulate the effect of a lesions growth).

In this way, using a motion vector field to locally shift the pixels/voxels, the synthetic image will more closely resemble a true image of blood vessels with lesions, as data about the bounds of the blood vessel will not be lost.

After this, the synthetic image may be generated by processing the modified image and the shape of each lesion in the synthesized set of one or more lesion. This process may, for example, comprise texturizing the image using a texture pattern to fill the shape of each synthetic lesion, i.e. at the location of the freed canvas that results from shifting the pixels/voxels of the image using the motion vector field.

In other words, step 140 may comprise using a motion vector field (defined by the determined shape and position of the synthetic lesions(s) to shift or modify pixels/voxels of the image. The shifting/modifying of the pixels/voxels can "free up" canvas space, at which a lesion is to be inserted. The free canvas space can be texturized using a herein described approach. Alternatively, the area of the image occupied by the motion vector field can be texturized using a described approach.

The skilled person will appreciate that training a machine-learning method requires training input data and training output data, which represents "ground-truth" annotations of the training input data. The proposed mechanism provides an approach for generating new examples of training input data. The corresponding training output data can be inferred from the synthetic lesion(s) generated and inserted into an image. For instance, the training output data may indicate the presence of a synthetic lesion, as well as characteristics of the synthetic lesion (e.g. its determined size and/or extent).

Accordingly, the method 100 may further comprise a step 150 of generating annotations for the synthetic image, based upon the characteristics of the set of one or more synthetic lesions in the synthetic image (e.g. identifying a presence, location, size, shape (e.g. outline), number, type, texture and so on of the synthetic lesion(s) in the synthetic image). The annotations may identify one or more characteristics of the lesions(s) in the synthetic image. The generated annotations may depend upon the desired (classification) process to be performed by the machine-learning method.

Generation of the annotations is made simple through the use of synthetic lesions, as the characteristics of the set of one or more synthetic lesions are known by virtue of their synthesis.

The annotation(s) may include any suitable data that could be provided by a machine-learning method. The data may comprise, for instance, a binary value, a categorical value and/or a continuous value.

A binary value may indicate the presence or absence of a synthetic lesion or a particular type of synthetic lesion or whether or not a number of synthetic lesions exceeds some predetermined threshold. Other suitable binary values will be readily apparent to the skilled person.

As another example, a categorical value is a value from a discrete set (i.e. finite) number of values. Each categorical value may, for instance, indicate one of a plurality of categories or types for the synthetic image. By way of example only, a categorical value may represent a type of lesion (or lesions) in a synthetic image (e.g. no lesion, calcification, wall-thickening and so on).

As yet another example, a continuous value may be a value indicating an extent of a (synthetic) lesion growth or presence within the synthetic image.

The skilled person would readily appreciate how the annotation(s) may be adapted for the purpose or intent of the machine-learning method, and that different machine-learning methods may be configured for performing different tasks (meaning that different annotations or ground truth answers need to be generated). An advantage of the present invention is that the generation of synthetic lesions means that synthetic annotations can be easily and readily produced based on the (known) characteristics of the set of one or more synthetic lesions.

It will be readily appreciated how a number of these steps may be repeated to generate additional synthetic images (and/or additional annotations for the synthetic images). For instance, steps 132 to 140/150 may be repeated (with slightly different parameters) to generate different synthetic images of the same synthetic lesion(s) at different growth stages. Steps 130 to 140/150 may be repeated (with different parameters) to generate different synthetic images of different synthetic lesion(s).

This approach facilitates the provision of a wide range of synthetic images representing different lesions and/or growth stages of lesions.

In some preferred examples, the step 140 of generating the synthetic image comprises only modifying a low-frequency component of the image 190 based on the synthesized set of one or more lesions, leaving the high-frequency component unchanged. This means that the synthesized image will share the same high-frequency component as the original image. This results in the synthesized image having a similar noise characteristic to the original image, so that the synthesized image more closely resembles a true or real-life image of one or more blood vessel(s) having one or more lesion(s) for improved training of the machine-learning method.

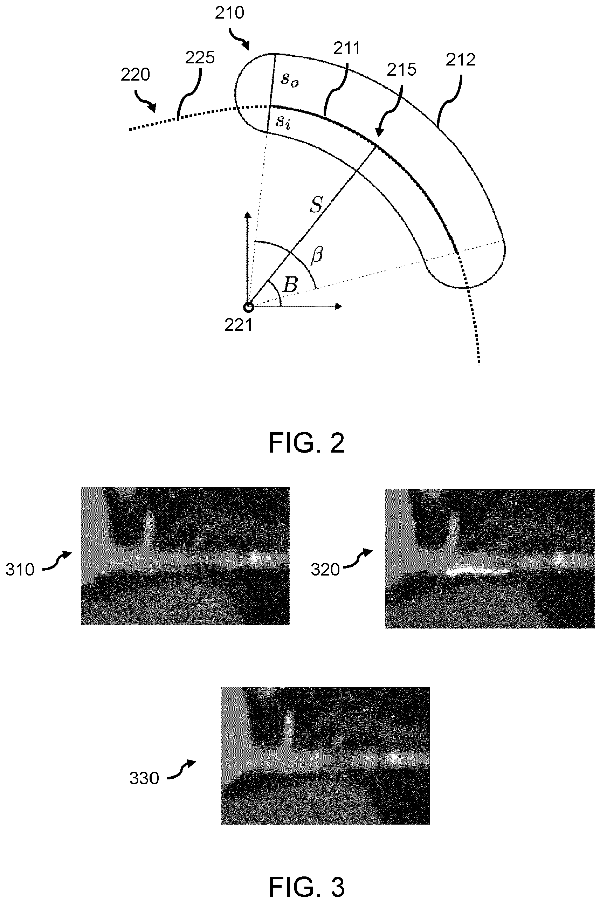

Figure 2 illustrates an example shape for a part of a lesion, as well as shape parameters for the lesion, and is used to describe an approach for synthesizing a lesion 210 according to an example embodiment. Thus, Figure 2 is used to describe an embodiment of steps 130 and 140.

Figure 2 represents a simple example in which a synthetic lesion 210 is to be inserted into or added to a cross-sectional view of a blood vessel 220 (whose exterior bounds 225 are partially illustrated). The centerline 221 of the blood vessel 220 is also illustrated. Conceptually, as this Figure illustrates a cross-section, it will be understood that the centerline 221 extends into and out of the page.

The shape of the lesion 210 is modelled, for the cross-sectional image, as a rounded annular segment/section, and is defined by shape parameters (e.g. including parameters analogous to height and width of an oblong). However, the skilled person would appreciate that a lesion can be modelled using other shapes, e.g. non-rounded annular segments/sections and/or irregular annular segments (e.g. annular segments of non-uniform thickness).

The shape of the lesion 210 is defined by first identifying or defining a location 215 for the lesion, e.g. a position with respect to the centerline 221 of a blood vessel 220 that lies near a wall/bound at which the lesion is to be grown/positioned. This location may be identified using a stochastic/pseudorandom selection process.

An exterior shape for the lesion is then generated by defining a number of (other) shape parameters with respect to the position of the vessel centerline. These (other) shape parameters may define an extent to which the lesion extends around the exterior bounds 225 of the blood vessel and/or a thickness of the lesion within the cross-sectional area.

The shape of the lesion 210 may therefore be defined by a first group of shape parameters, which define the position 215 of the lesion with respect to the centerline 221 of the blood vessel 220, and a second group of shape parameters, which define the size and shape of the lesion 210 with respect to the determined position.

The first group of shape parameters may include a major radius S, which defines a distance between (a position on) the vessel centerline and a position on the centerline of the lesion. The length of the major radius S is defined by the segmentation of the blood vessel, as it is equal to a distance between the centerline 221 of the blood vessel and the exterior bounds 255 of the blood vessel, i.e. as the lesion is positioned on the exterior bounds 255 of the blood vessel.

The first group of shape parameters may also include an orientation angle B. The orientation angle B defines a position of the lesion 211 about an axis defined by the centerline.

The location of the lesion 210 may be set by, for example, selecting a value for the orientation angle B using a pseudorandom or stochastic selection process. The value of the major radius can then be defined from the segmentation of the blood vessel, as the segmentation results will define a distance between the centerline 221 of the blood vessel 220 and the exterior bounds 225 of the blood vessel.

The second group of shape parameters may include an opening angle β. The opening value defines an angular range of (the centerline 211 of) the lesion, i.e. the amount of a circle taken up by the lesion or the arc measure. The centerline 211 of the lesion is thereby defined as a line having a center at the position defined by the first group of shape parameters, and having an arc with an arc measure defined by the opening angle. The "center" of the arc of the centerline 211 is the centerline 221 of the blood vessel 220. This results in the synthetic lesion thereby follows the exterior bounds 225 of the blood vessel 220, as at a cross-section of a blood vessel, a blood vessel can be assumed or modelled to be generally circular.

For the sake of further explanation, it is noted that an opening angle having a value of 0° would indicate no synthetic lesion, a value of 90° would indicate that a fourth of a circle is covered by a synthetic lesion and a value of 360° would indicate that the synthetic lesion goes around an entire whole circle.

The second group of shape parameters may also include an inner minor radius sᵢ, which defines the distance between the centerline 211 of the lesion and a side of the lesion closest to the centerline 221 of the blood vessel 220 along the orientation angle B, and an outer minor radius sₒ, which defines the distance between the centerline 211 of the lesion 210 and a side of the lesion 210 further from the centerline 221 of the blood vessel 220 along the orientation angle. The edge of the lesion shape may be rounded based on the inner and outer minor radius.

In some examples, although less preferred, the inner minor radius and the outer minor radius may be replaced by a single radius value that defines the thickness of the lesion 211. However, use of the minor and major radii is preferred because this allows for closer simulation of true or real-life lesions (which tend to extend outside of the blood vessel to a greater extent than inside the blood vessel.

The above described shape parameters are suitable for a single cross-sectional image, where is can be assumed that a lesion can be simulated using a thickened arc or annulus sector/segment (as illustrated). In more complex procedures, e.g. with 3D images, a 3D lesion may be defined using multiple sets of the above described shape parameters, each representing a shape of part of the lesion at different positions (and therefore different cross-sections) along the centerline of a blood vessel. In this instance, an additional shape parameter may be provided to indicate the relative position along a centerline of the part of the lesion. The overall 3D lesion can therefore be built up from multiple lesion portions, each defined with the shape parameters previously described with reference to Figure 2.

The values for shape parameters (excluding those set/defined by the segmentation) may be assigned using a pseudorandom or stochastic selection process, e.g. wherein the upper and lower bounds for possible values of the shape parameters are defined from shape parameters of real lesions (e.g. from literature or expert clinician input). In some examples, the upper and lower bounds for possible values of the shape parameters may be set by a user (e.g. if there is considered to be insufficient data for training a machine-learning method at particular values) and/or by a system operating a machine-learning method itself (e.g. if it is determined that an error-rate of a machine-learning method is too great for particular shape parameters).

From the shape, it is possible to generate a motion vector field that mimics the lesion growth process. In particular, a motion vector field can be defined for the image that simulates the growth of the lesion from the centerline 211 to the exterior bounds 212.

By way of example, the motion vector field may a vector field having values set to zero at the centerline 211 of the synthetic lesion 210 and outside the exterior bounds 212 of the synthetic lesion. The motion vectors (in the motion vector field) may have a direction perpendicular/normal to the exterior bounds of the synthetic lesion (as well as the centerline 211). Within the bounds 212 of the lesion, the magnitude of the motion vectors of the motion vector field may be modulated to smoothly vary from the centerline 211 towards the target shape boundary 212.

Figure 3 illustrates a result of a performing a method according to an embodiment. In particular, Figure 3 illustrates synthesized images for different types of texture for a synthesized lesion (which is generated using the approach described in Figure 1). It is noted that Figure 3 depicts the stretched blood vessel along the coronary centerline, rather than a cross-sectional area as illustrated in Figure 2.

A first image 310 depicts a result of applying a first texture to a generated lesion when combining the generated lesion and the image, the first texture being a "wall thickening" texture to model a wall thickening of a blood vessel.

A second image 320 depicts a result of applying a second texture to a generated lesion when combining the generated lesion and the image, the second texture being a "calcification" texture to model a calcification of a blood vessel.

A third image 330 depicts a results of applying a third texture to a generated lesion when combining the generated lesion and the image, the third texture being a "spotty calcification" texture to model a partial calcification of a blood vessel.

In this way, it will be clear that different textures may be applied to a generated lesion, when combining the generated lesion and the image, to generate the synthetic image appropriate for a desired machine-learning task. The type of texture applied to the generated lesion may be used to define annotations for the synthesized image (e.g. annotations for ground-truth data).

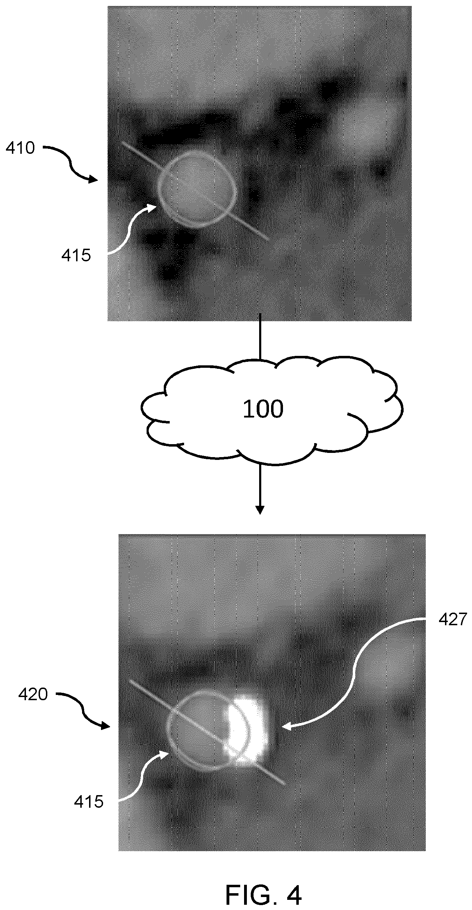

Figure 4 illustrates another result of performing a method according to an embodiment of the invention. In particular, Figure 4 illustrated an original image and a synthesized image (generated from the original image), which is generated using the method 100 described in Figure 1.

The original image 410 illustrates a cross-section of a blood vessel 415, which has undergone image segmentation (to identify a shape of the blood vessel as illustrated). Thus, Figure 4 illustrates a cross-sectional area of the blood vessel, in a similar manner to Figure 2.

The synthesized image 420 illustrates the same cross-section of the blood vessel 415, with a synthesized lesion 427 having been added to the image. Due to use of the motion vector field, the pixel/voxel values of the areas surrounding the synthesized lesion more closely resemble the real-life effect of a lesions growth (e.g. rather than simply superimposing a lesion over an blood vessel image).

The present disclosure proposes an approach for generating training data for a machine-learning algorithm, and in particular a machine-learning algorithm for processing an image for assessing lesions in blood vessels of the image.

A machine-learning algorithm is any self-training algorithm that processes input data in order to produce or predict output data. Here, the input data comprises an image of the subject and the output data comprises characteristics of blood vessel lesions of the subject. These characteristics may include a presence (or absence) of one or more lesion(s), a location of any lesions, a shape/size of any lesions, a type of any lesions and so on.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

As previously explained, the present disclosure relates to approaches for generating a training dataset for the machine-learning algorithm. In particular, the training input data entries correspond to example synthesized images. The training output data entries correspond to characteristics of lesions in the synthesized images.

In some examples, the machine-learning method algorithm is configured to receive additional data as input for determining characteristics of lesions in the image. For instance, the machine-learning method may receive metadata of the image, e.g. characteristics of the mechanism used to obtain the image, such as an imaging type, an imaging modality and so on.

In this scenario, when generating the synthesized image(s), metadata of the original image may be associated with the synthesized image(s) for use in subsequent training of the machine-learning algorithm. In particular, the metadata of the original image used to generate the synthesized image(s) may become the metadata for the synthesized image.

The approach suggested by the present disclosure facilitates the generation of synthetic images of blood vessels (having lesions) in a region of interest of the patient. The region of interest can be selected through selection of appropriate image(s) for the region of interest. This means that site-specific imaging aspects can be taken into account during the generation of synthetic images, to improve the relevance of generated synthetic images.

Besides the aforementioned examples of lesions (wall thickening, calcification and/or partial calcification), the proposed methodology could be employed to generate other types of lesions, growths or blockages, such as carotid plaque, aneurysms, clots and so on. Similarly, the region of interest may vary depending upon the use case scenario, e.g. the region of interest may be the brain, the heart, the coronary artery, veins (e.g. for analysis of deep vein thrombosis) and so on.

Training of a machine-learning method may make use of user feedback, e.g. indicating where a prediction by the machine-learning method is incorrect, to improve the accuracy of the machine-learning method. Mechanisms for employing user feedback in this manner are known in the art.

In some examples, the proposed method for generating synthetic images could be (automatically) employed if a machine-learning algorithm makes more errors than desired.

Thus, in some embodiments, the process for generating synthetic images (for the machine-learning method) is (automatically) triggered in response to an accuracy of the machine-learning method falling below some predetermined threshold, or the error rate of the machine-learning method rising above some predetermined error threshold. This facilitates directed and on-site training for a machine-learning method to automatically improve the accuracy of machine-learning methods without wasting processing power generating additional synthetic images for sufficiently accurate machine-learning methods.

The proposed approach for generating synthetic images allows for control over the synthesis of lesions within a synthetic image (e.g. by setting the bounds of parameters for lesions in step 130-140. This can be performed automatically (e.g. if there is insufficient data for a particular area or type of lesion) or responsive to a user input, as previously described. For instance, parameter ranges can be specified where weaknesses are observed and new synthetic images can be generated to retrain the machine-learning method.

In some examples, a process of training a machine-learning method comprises obtaining feedback from a user concerning an (in)correct prediction by the machine-learning method and using this feedback to generate a new training data entry (e.g. by generating an annotation from feedback from the user (as a ground-truth annotation) and an input image as the image that prompted the user feedback.

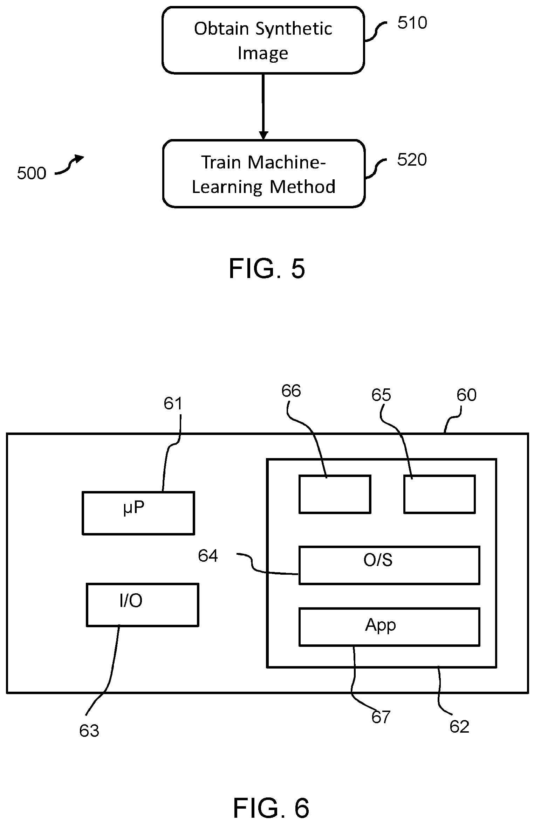

Figure 5 illustrates a computer-implemented method for training a machine-learning method for assessing lesions in blood vessels of an image,
The computer-implemented method 500 comprises a step 510 of obtaining synthetic image(s). The synthetic image(s) is/are obtained using a previously described approach. The synthetic image(s) may be supplemented with real image(s), e.g. of blood vessels with lesions.

The step 510 also obtains one or more annotations for the synthetic image(s), such as those generated in previously described embodiments. The annotation(s) facilitate accurate training of the machine-learning method, by providing suitable ground-truth examples of the output of the machine-learning method. Of course, any real images are also accompanied with annotations.

The computer-implemented method 500 further comprises a step 520 of training a machine-learning method for assessing lesions in blood vessels of an image using the obtained synthetic image(s). Step 520 may use any suitable learning technique for training the machine-learning method, which are well-known in the art. Examples include supervised learning techniques.

By way of further example, Figure 6 illustrates an example of a processing system 60 (e.g. a computer) within which one or more parts of an embodiment may be employed. Various operations discussed above may utilize the capabilities of the processing system 60. For example, one or more parts of a system for generating the synthetic image (or training a machine-learning method) may be incorporated in any element, module, application, and/or component discussed herein. In this regard, it is to be understood that system functional blocks can run on a single processing system or may be distributed over several processing systems and locations (e.g. connected via internet).

The processing system 60 includes, but is not limited to, PCs, workstations, laptops, PDAs, palm devices, servers, storages, and the like. Generally, in terms of hardware architecture, the processing system 60 may include one or more processors 61, memory 62, and one or more I/O devices 67 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 61 is a hardware device for executing software that can be stored in the memory 62. The processor 61 can be virtually any custom made or commercially available processor, a central processing unit (CPU), a digital signal processor (DSP), or an auxiliary processor among several processors associated with the processing system 60, and the processor 61 may be a semiconductor based microprocessor (in the form of a microchip) or a microprocessor.

The memory 62 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 62 may incorporate electronic, magnetic, optical, and/or other types of storage media. Note that the memory 62 can have a distributed architecture, where various components are situated remote from one another, but can be accessed by the processor 61.

The software in the memory 62 may include one or more separate programs, each of which comprises an ordered listing of executable instructions for implementing logical functions. The software in the memory 62 includes a suitable operating system (O/S) 65, compiler 64, source code 63, and one or more applications 66 in accordance with exemplary embodiments. As illustrated, the application 66 comprises numerous functional components for implementing the features and operations of the exemplary embodiments. The application 66 of the processing system 60 may represent various applications, computational units, logic, functional units, processes, operations, virtual entities, and/or modules in accordance with exemplary embodiments, but the application 66 is not meant to be a limitation.

The operating system 65 controls the execution of other processing system programs, and provides scheduling, input-output control, file and data management, memory management, and communication control and related services. It is contemplated by the inventors that the application 66 for implementing exemplary embodiments may be applicable on all commercially available operating systems.

Application 66 may be a source program, executable program (object code), script, or any other entity comprising a set of instructions to be performed. When a source program, then the program is usually translated via a compiler (such as the compiler 64), assembler, interpreter, or the like, which may or may not be included within the memory 62, so as to operate properly in connection with the O/S 65. Furthermore, the application 66 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 67 may include input devices such as, for example but not limited to, a mouse, keyboard, scanner, microphone, camera, etc. Furthermore, the I/O devices 67 may also include output devices, for example but not limited to a printer, display, etc. Finally, the I/O devices 67 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 67 also include components for communicating over various networks, such as the Internet or intranet.

If the processing system 60 is a PC, workstation, intelligent device or the like, the software in the memory 62 may further include a basic input output system (BIOS) (omitted for simplicity). The BIOS is a set of essential software routines that initialize and test hardware at startup, start the O/S 65, and support the transfer of data among the hardware devices. The BIOS is stored in some type of read-only-memory, such as ROM, PROM, EPROM, EEPROM or the like, so that the BIOS can be executed when the processing system 60 is activated.

When the processing system 60 is in operation, the processor 61 is configured to execute software stored within the memory 62, to communicate data to and from the memory 62, and to generally control operations of the processing system 60 pursuant to the software. The application 66 and the O/S 65 are read, in whole or in part, by the processor 61, perhaps buffered within the processor 61, and then executed.

When the application 66 is implemented in software it should be noted that the application 66 can be stored on virtually any processing system readable medium for use by or in connection with any processing system related system or method. In the context of this document, a processing system readable medium may be an electronic, magnetic, optical, or other physical device or means that can contain or store a processing system program for use by or in connection with a processing system related system or method.

The application 66 can be embodied in any processing system-readable medium for use by or in connection with an instruction execution system, apparatus, or device, such as a processing system-based system, processor-containing system, or other system that can fetch the instructions from the instruction execution system, apparatus, or device and execute the instructions. In the context of this document, a "processing system-readable medium" can be any means that can store, communicate, propagate, or transport the program for use by or in connection with the instruction execution system, apparatus, or device. The processing system readable medium can be, for example but not limited to, an electronic, magnetic, optical, electromagnetic, infrared, or semiconductor system, apparatus, device, or propagation medium.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims. A processing system program may be stored/distributed on a suitable medium, such as an optical storage medium or a solid-state medium supplied together with or as part of other hardware, but may also be distributed in other forms, such as via the Internet or other wired or wireless telecommunication systems.

The mere fact that certain measures are recited in mutually different dependent claims does not indicate that a combination of these measures cannot be used to advantage. If the term "adapted to" is used in the claims or description, it is noted the term "adapted to" is intended to be equivalent to the term "configured to". Any reference signs in the claims should not be construed as limiting the scope.

## Claims

1. A computer-implemented method (100) for generating a synthetic image (310, 320, 330, 420) for training a machine-learning method for assessing lesions in blood vessels (415) of an image (190, 410), the computer-implemented method comprising:
obtaining (110) an image (190, 410) of blood vessels in a region of interest of a subject;
performing (120) image segmentation on the image to identify one or more parameters of the blood vessels from the image;
synthesizing (130) a set of one or more lesions (427) for one or more of the blood vessels of the image by processing the identified one or more parameters of the blood vessels;
generating (140) the synthetic image (310, 320, 330, 420), for training a machine-learning method for assessing lesions in blood vessels of an image, by combining the image and the synthesized set of one or more lesions for one or more of the blood vessels contained in the image; and
generating (150) one or more annotations for the synthetic image, based upon the characteristics of the set of one or more synthetic lesions in the synthetic image.

2. The computer-implemented method (100) of claim 1, wherein the image (190, 410) comprises a computed tomography image.

3. The computer-implemented method (100) of any of claims 1 or 2, wherein the one or more parameters of the blood vessels includes a position and/or shape of at least one blood vessel in the image.

4. The computer-implemented method (100) of any of claims 1 to 3, wherein:
the one or more parameters of the blood vessels includes at least a position of at least one blood vessel in the image; and
the step (130) of synthesizing a set of one or more lesions comprises defining a location for each lesion, with respect to the image, based on the identified position of at least one blood vessel in the image.

5. The computer-implemented method (100) of claim 4, wherein the step of defining a location for each lesion comprises defining the location of each lesion using a partially stochastic generation procedure.

6. The computer-implemented method (100) of any of claims 1 to 5, wherein:
the one or more parameters of the blood vessels includes at least a shape of at least one blood vessel in the image; and
the step (130) of synthesizing a set of one or more lesions comprises defining a shape for each lesion based on the identified shape of the at least one blood vessel in the image.

7. The computer-implemented method (100) of claim 6, when dependent on any of claims 4 or 5, wherein the step (140) of generating the synthetic image comprises:
generating, for each lesion, a motion vector field, which imitates the growth of the respective lesion, based on parameters of the shape of the respective lesion; and
generating the synthetic image by processing the image, the shape and the motion vector field for each lesion in the synthesized set of one or more lesion.

8. The computer-implemented method (100) of claim 7, wherein the step (140) of generating the synthetic image comprises:
using the motion vector field, of each lesion in the synthesized set of one or more lesion, to modify the values of pixels or voxels of the image at the position of each of the synthesized set of one or more lesions, to thereby generate a modified image; and
generating the synthetic image by processing the modified image and the shape of each lesion in the synthesized set of one or more lesion

9. The computer-implemented method (100) of claim 8, wherein the step of using the motion vector field to modify the values of pixels or voxels comprises, for each motion vector field, locally shifting the pixels or voxels of the image at the position of the motion vector field on values of the motion vector field.

10. The computer-implemented method (100) of claim 9, wherein the step of generating the synthetic image comprises texturizing the synthesized set of one or more lesions using a texture pattern.

11. The computer-implemented method (100) of any of claims 1 to 10, wherein the blood vessels (145) are coronary arteries.

12. A computer-implemented method (500) of training a machine-learning method for assessing lesions in blood vessels of an image, the computer-implemented method comprising:
obtaining (510) one or more synthetic images, and one or more annotations for each synthetic image, generated using the method of any one or claims 1 to 11; and
training (520) a machine-learning method for assessing lesions in blood vessels of an image using the obtained one or more synthetic images.

13. A processing system program product comprising computer program code means which, when executed on a computing device having a processing system, causes the processing system to perform all of the steps of the method according to any of claims 1 to 12.

14. A processing system (60) for generating a synthetic image (310, 320, 330, 420) for training a machine-learning method for assessing lesions in blood vessels (415) of an image (190, 410), the processing system being configured to:
obtain (110) an image of blood vessels in a region of interest of a subject;
perform (120) image segmentation on the image to identify one or more parameters of the blood vessels from the image;
synthesize (130) a set of one or more lesions for one or more of the blood vessels of the image by processing the identified one or more parameters of the blood vessels;
generate (140) the synthetic image, for training a machine-learning method for assessing lesions in blood vessels of an image, by combining the image and the synthesized set of one or more lesions for one or more of the blood vessels contained in the image;
generate (150) one or more annotations for the synthetic image, based upon the characteristics of the set of one or more synthetic lesions in the synthetic image.

15. A processing system (60) for training a machine-learning method for assessing lesions in blood vessels (415) of an image (190, 410), the processing system being configured to:
obtain (510) one or more synthetic images, and one or more annotations for each synthetic image, generated using the method of any one or claims 1 to 11; and
train (520) a machine-learning method for assessing lesions in blood vessels of the image using the obtained synthetic image.
